# EUROPEAN PATENT APPLICATION

(11) **EP 3 187 220 A1**
(43) Date of publication of application: **05.07.2017**
(21) Application number: 16183587.1
(22) Date of filing: 10.08.2016
(51) Int. Cl.: A61M 15/00, A61M 16/20, A61M 15/08, A61M 11/02

(54) **BREATHE CLEAR**

(30) Priority: 04.01.2016 US 201614987168
(71) Applicant: Renaud, John, Tinley Park, IL 60477 (US); Hayes, John, Tinley Park, IL 60477 (US)
(72) Inventor: Renaud, John, Tinley Park, IL 60477 (US); Hayes, John, Tinley Park, IL 60477 (US)
(74) Representative: Romano, Giuseppe

(57) **Abstract**

The present invention provides an inhalation device for controlled delivery of medicament to the lungs of the user. The inhalation device comprises an elongated housing enclosing a mouthpiece/nasal-piece in fluid communication to a narrow cylindrical tube, the narrow cylindrical tube having diameter substantially similar to the lumen diameter of human bronchi. The other end of narrow cylindrical tube is fluidly connected to a reservoir containing the medicament suspended or dissolved in the propellant. The flow of propellant from reservoir to the narrow cylindrical tube is regulated by a one-way valve, which could be electrical/electronic or a mechanical valve. The invention is advantageous by providing positive airway pressure to keep the constricted air passages of the user open, which is combined, with laminar flow of medicament suspended/dissolved in the propellant, thus, resulting in efficient deposition of medicament to the target tissues of the lungs.

## Description

### FIELD OF INVENTION

The present invention generally relates to inhalers for controlled delivery of medicament, and in particular to inhalers providing a combination of positive airway pressure and laminar flow of suspended medicament.

### BACKGROUND OF INVENTION

The use of inhalers of known designs and configurations for controlled delivery of medicament is well known in the prior art. More specifically, hand-held pressurized metered-dose inhalers (pMDIs) are generally used to deliver anti-inflammatory and bronchodilator medicaments to the lungs for treatment of asthma and chronic obstructive pulmonary diseases (COPD). The mechanism behind working of pressurized metered-dose inhalers is a propellant under pressure causes a metered dose of a drug to eject from a valve and into a spacer wherein an aerosol plume is formed which could be then inhaled by the user.

Prior arts disclose number of designs and configurations of metered dose inhalers including a U.S. Patent No. 5,544,647 issued to Iep Group, Inc. issued on Aug 13, 1996, which discloses a metered dose inhaler having an improved electronic counting means to indicate the doses remaining in the aerosol canister component of the inhaler assembly. Another, U.S. Patent No. 6,708,688 issued to Darren Rubin et.al. issued on March 23, 2004 discloses a metered dosage inhaler system with variable positive pressure settings for providing medication and exercise to the lungs. Yet, another U.S. Patent No. 7,900,625 issued to North Carolina State University, issued on March 8, 2011 discloses a smart inhaler system comprising an inhalation device for directed aerosol delivery facilitated by an adaptive nozzle and a mechanism for inhalation waveform modulation is provided. Methods of using the smart inhaler system for delivering an active agent to a target area of a lung of a subject are further provided.

However, the inhalers of prior art suffers from one or more limitations, and in particular the pressurized inhalation device of prior art produces a plume of medicament suspended in a propellant, which the user has to inhale. The drug deposition into lungs efficiency of such system is usually poor and depends upon inhalation power of the user, which may vary from person to person and may further depend upon medical condition of the user. Thus, a need could be appreciated for an inhaler that could generate positive airway pressure to keep the air passage of the user open for better deposition of drug into the lungs.

### SUMMARY OF THE INVENTION

The present invention, therefore, has as its principal object to provide an inhalation device for delivering a medicament to the target area of the lungs.

Another object of present invention is to provide a method for delivering a medicament to the lungs.

An additional object of the present invention is that the inhalation device provides positive airway pressure to keep the constricted air passages of the user open for delivering the medicament to the lungs.

Another object of the present invention is that the inhalation device provides a laminar flow of medicament suspended in the propellant for efficient deposition of medicament into the lungs.

A further object of present invention is that inhalation device permits variable positive pressure adjustments.

Yet another object of present invention is that the inhalation device is economic in manufacture and easy to use.

Certain embodiments of present invention provide an inhalation device for controlled delivery of medicament to the lungs of the user. The device is advantageous by providing positive airway pressure to keep the constricted air passage of the user open, combined with laminar flow of medicament suspended/dissolved in the propellant, which results in efficient deposition of medicament to the target tissues in the lungs.

In one embodiment, the inhalation device comprises an elongated housing, a mouthpiece/nasal-piece of substantially conical shape with narrow mouth of the mouthpiece/nasal piece juxtaposed over the open end of a cylindrical tube and the wider mouth of mouthpiece/nasal piece coupled to the housing. The other end of narrow cylindrical tube is fluidly connected to a reservoir containing a medicament suspended or dissolved in a propellant. The narrow cylindrical tubes have a diameter substantially similar to the lumen diameter of human bronchi, the bronchi referred hereinafter refers to primary bronchi. The reservoir, apart from the medicament and propellant, may further contain surfactants and solvent modifiers approved for inhaler formulations to manipulate the suspension or solvation of medicament in the propellant. The flow of propellant from the reservoir to the narrow cylindrical tube is regulated using a calibrated one-way valve wherein the actuation of valve causes a metered portion of the reservoir content to be released, whereby the pressure of the propellant carries the dissolved, or suspended medicament into the narrow cylindrical tube. Moreover, the valve could be configured to allow changing the pressure of propellant released from the valve into the narrow cylindrical tube.

In another embodiment, the one-way valve could be an electrical/electronic one-way valve that could be controlled using a microprocessor or electrical current or the one-way valve could be mechanical valve that could be mechanically actuated. The inhalation device having the electrical valve could further comprises a control unit operably coupled to the valve, to regulate the pressure and volume of propellant released from the valve opening. The control unit includes a microprocessor, a means to adjust the desired pressure of propellant, and a means to power the control unit. The control unit may further comprise a display screen showing the pre-set pressure.

In another embodiment, the inhalation device having a mechanical valve further comprise a mechanical unit operably coupled to the valve, to regulate the pressure and volume of propellant released from the valve opening. The mechanical unit could include a rotatory dial having different pressure settings.

In another embodiment, the propellant with dissolved or suspended medicament under pressure released from the valve orifice passes through the narrow cylindrical tube, and while passing through the length of the cylindrical tube, the turbulence of medicament is decreased and the flow of propellant having suspended/dissolved medicament becomes more laminar.

In another embodiment, the device could be produced in compact size that is portable and could be carried in a pocket or purse. Compact size and portability allows the device to be easily carried and reached when required, in particular during a life threatening condition.

In another embodiment, the present invention provides the inhalation device that has a replaceable and interchangeable reservoir. The same inhalation device could be used for multiple purposes such as delivering different medicament like a bronchodilator, anti-inflammatory, or vaccine. The user could simply replace the reservoir containing the desired medicament for inhalation. The device could be used for both self-administration or with assistance of other person.

In addition to the various objects and advantages of the present invention described with some degree of specificity above it should be obvious that additional objects and advantages of the present invention will become more readily apparent to those persons who are skilled in the relevant art from the following more detailed description of the invention.

### BRIEF DESCRIPTION OF THE DRAWINGS

To further clarify various aspects of some example embodiments of the present invention, a more particular description of the invention will be rendered by reference to specific embodiments thereof, which are illustrated in the appended drawing. It is appreciated that the drawing depicts only illustrated embodiments of the invention and are therefore not to be considered limiting of its scope. The invention will be described and explained with additional specificity and detail through the use of the accompanying drawing in which:
Fig. 1 is the perspective view of inhalation device showing the mouthpiece; narrow cylindrical tube; reservoir and control unit.
Fig. 2 is the exploded view of the inhalation device of Fig. 1.

### DETAIL DESCRIPTION OF THE INVENTION

The present invention provides an inhalation device with positive airway pressure and laminar flow of medicaments providing efficient delivery of the medicament to the target tissues of the lung.

Before explaining at least one embodiment of the invention in detail, it is to be understood that the invention is not limited in its application to the details of construction and the arrangements of the components set forth in the following description. The invention is capable of other embodiments and of being practiced and carried out in various ways. Also it is to be understood that the phraseology and terminology employed herein are for the purpose of description and should not be regarded as limiting.

As used in this specification and the appended claims, the singular forms "a", "an", and "the" include plural referents unless the content clearly dictates otherwise.

Unless otherwise defined, all terms (including technical and scientific terms) used herein have the same meaning as commonly understood by one having ordinary skill in the art to which this invention belongs. It will be further understood that terms, such as those defined in commonly used dictionaries, should be interpreted as having a meaning that is consistent with their meaning in the context of the relevant art and the present disclosure and will not be interpreted in an idealized or overly formal sense unless expressly so defined herein.

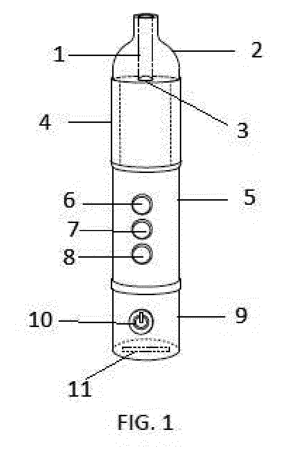

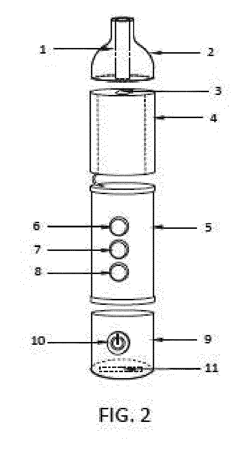

Now, referring to the **Figs. 1** **&** **2****,** that shows the inhalation device according to present invention. The device as shown in the figure is an elongated cylindrical housing that measures approx. 4 to 5 inches in length and ½ to ¾ inches in diameter. The device could be fabricated from material ordinary used in making medical devices, such as plastics, light weigh metals, or alloys. Preferably, the inhalation device could be made from plastic or lightweight aluminum alloy. It could be advantageous to produce a disposable inhalation device using plastic material and a re-usable inhalation device from sturdy material such as an aluminum alloy. The elongated cylindrical tube includes multiple units or modules arranged lengthwise of the cylindrical housing, wherein the individual module may or may not be separated from other module. The inhalation device includes a narrow cylindrical tube **1** that is connected to the reservoir **4.** A mouthpiece **2** covers the narrow cylindrical tube and is configured to be hold by the lips of user. The diameter of mouthpiece tapers in front and terminates such as a portion of narrow cylindrical tube protrudes from the mouthpiece providing laminal flow. The reservoir holds the medicament suspended or dissolved in the propellant. The propellant could be any propellant approved for use in inhalation devices. The reservoir may further contain surfactants and solvent modifiers known to skill person for use in inhalation devices for modifying the properties of propellant, such that to improve the suspension and/or solvation of medicament in the propellant.

The flow of propellant from the reservoir to the narrow cylindrical tube is regulated by a one-way valve **3** which when actuated allows the propellant under pressure to carry the medicament suspended or dissolved in the propellant into the narrow cylindrical tube. The valve is configured to release a metered dose of the medicament. Furthermore, the valve could be an electrical/electronic valve or a mechanical valve. The device shown in **Fig. 1** includes the electrical one-way valve, which is operably coupled to the control unit **5,** that regulates the opening of the valve and the pressure of the propellant released from the valve. The control unit could be powered by a power module **9.** The power module includes a means to power the control unit and the valves assembly. The power module includes a battery of sufficient capacity to power the control unit and valve assembly of the inhaling device, such that the device could be used multiple times. The batteries could be replaceable, and in such case, the power module could be separated from rest assembly exposing the battery, which could be then replaced. In case the inhalation device is produced for single use or is disposable, the battery power module may not be separable for replacing the batteries. Furthermore, rechargeable batteries could be provided for re-usable type inhalation device. In case, the rechargeable batteries are provided, appropriate recharging apparatus could also be provided that includes an AC to DC adaptor, and a charging port **11** protruding from the power module for connecting the recharging apparatus. The charging port could be a normal or mini USB port **11** and recharging apparatus could be a wall mountable adaptor with an extending cord for connecting to the recharging port of the power module. The power module could further include a power button **10,** which may be used to turn the inhalation device on and off.

The control unit **5** could digitally regulate the pressure and amount of medicine released with each medicament. The device may allow the user to incrementally manipulate the positive airway pressure generated according to the requirement or medical condition of the user. **Fig.1** shows three buttons **6, 7, & 8** corresponding to different levels of airway pressure. Button **6** may provide minimum positive airway pressure followed by button **7** providing incremental increase in pressure and could be suitable for normal use and the last button **8** may provide highest positive pressure. The user could choose the level of positive pressure appropriate to their medical condition, for example, normal pressure setting could be suitable for user who could take deep breaths, and maximum pressure setting could be suitable for users whose air passage is constricted due to a medical condition and could not take deep breathe.

Besides the provision of three level pressure resistances as shown in **Fig. 1****,** the device may include multiple levels of pressure adjustments by the user. The device may be provided with two up and down button, wherein the up button could be used for incremental increase the positive pressure and the down button could be used for incremental decrease the positive pressure. Optionally, the device may further be provided with a display screen for displaying the pressure and other details like battery status, medicament status, propellant status etc.

Unlike, the digital operation, the inhaling device could also be manually operated wherein the device includes mechanical valve operably coupled to a rotary dial, instead of power module and control unit. The rotary dial provides for different pressure settings and the dial could be operably coupled to the mechanically actuated valve controlling the release of propellant. For example, the dial could be rotated between three pressure resistance levels, low, mid and high. Alternatively, the dial could be provided with a scale such as from 1-9, wherein 1 correspond to lowest pressure and the pressure could be gradual and incrementally increase from 1 to 9, and 9 having maximum possible positive airway pressure provided by the inhalation device.

Furthermore, it is to be noted that the functioning of electrical or mechanical valves and movement of the actuator to actuate the valve within the scope of the present invention will be apparent to one of ordinary skill in the art in view of the present disclosure.

The inhalation device is advantageous by providing laminar airflow, which directs the concentrated stream of medicament under positive pressure to deposit the medicament in the tissues of the lung. The laminal airflow is provided with a narrow cylindrical tube connecting the mouthpiece to the medicament reservoir. The diameter of the narrow cylindrical tube could be substantially similar to the lumen diameter of the human bronchi, thus ensuring efficient delivery of the medicament through the air passages. The device shown in the **Fig. 1** is having the diameter of approximately 1 cm, which provides a concentrated stream of medicament having laminar flow characteristics. It is obvious that the size of the narrow cylindrical tube could be varied to adjust to the size of human bronchi.

Although, the inhalation device shown in the **Fig. 1** is having a mouthpiece, the inhalation device could be adapted for both nasal inhalation and oral inhalation and appropriately the inhalation device could be provided with a nasal piece or a mouthpiece respectively. The nasal piece is configured to hold the device against the nasal opening with a front narrow portion of nasal piece partially inserted into the nasal opening. The nasal piece provides sealing against the nasal opening preventing escape of the medicament. The inhalation device further includes a push button to actuate the inhalation device for releasing the medicament suspended in the propellant and at the pre-set pressure conditions.

The positive airway pressure helps to keep the constricted air passages of the user open for delivering the concentrated stream of medicament to the target areas in the lung. The positive airway pressure is particularly useful for delivering the mist of medicine deep into the airways and the lungs, even when the user is unable to take a deep breath. The propellant gasses could deliver the medication in a laminar flow that in combination with positive pressure i.e. greater pressure that exists within the lungs and airways, could efficiently reach the target tissues of the lung. Thus, rather than attempting to deeply inhale a puff form from a conventional inhaler, the present invention could, through the inhalation device, direct the needed medication deeply into the bronchial airways and lungs. The inhalation device of present invention could be an ideal inhaler for asthma suffers, as well as for individuals with COPD, emphysema, and other respiratory conditions and illness. Further, the inhalation device could be used for delivering a variety of medicines in aerosolized form, wherein such medications may be effectively administered through respiratory/pulmonary systems. Such medicines may include cough suppressants, anti-histamines, fever suppressants, and so forth.

The device could be used by the user for delivering the medicament in any of the obvious manner, for example, the user selects the pressure level that corresponds with their ability to inhale. The user could then, exhale and then place inhalation device in their mouth and thereafter actuates the push button to release the medicament suspended/dissolved in propellant under positive pressure and simultaneously inhaling the released stream as deeply as possible. The user may then re-adjust the pressure from minimum up to maximum depending on their ability to inhale as deeply as possible and repeat the process of inhalation if necessary.

The user could easily coordinate between the actuation of push button to release the medicament and inhalation of the released medicament. If the user finds it is very difficult to inhale, they could use maximum pressure to attempt to force the medication into the bronchi to alleviate the bronchial spasm. Also more than one attempt may be required depending upon the medical condition of the user.

It is to be understood that the inhalation device encompasses a variety of alternatives. For example, the elongated housing of inhalation device could be round to square in shape. Moreover, the inhalation device could be made for single use or multiple uses. Furthermore, the inhalation device could have a replaceable reservoir, or the inhalation device could be disposable. In addition, the inhalation device could be used to deliver most types of medicaments formulated to deliver to lungs through inhalation, for example, bronchodilators anti-inflammatory drugs, vaccines, and diabetes drugs. Also, the inhalation device could be made as water resistant and shock resistant.

Those skilled in the art should appreciate that they can readily use the disclosed conception and specific embodiment as a basis for designing or modifying other structures for carrying out the same purposes of the present invention and that such other structures do not depart from the spirit and scope of the invention in its broadest form.

## Claims

1. An inhalation device for providing a medicament to the lungs of a patient, said medicament dissolved or suspended in a propellant, said inhalation device comprising:
a. a cylindrical tube in fluid communication with a reservoir and configured to deliver a substantially laminar flow of said medicament dissolved or suspended in said propellant from said reservoir to oral or nasal cavity of said user, said cylindrical tube having a diameter substantially similar to the lumen diameter of a human bronchi;
b. means to provide said medicament dissolved or suspended in said propellant under positive airway pressure from said reservoir.

2. The inhalation device of claim 1, wherein said reservoir contains said medicament dissolved or suspended in said propellant under pressure.

3. The inhalation device of claim 1, wherein said reservoir is replaceable.

4. The inhalation device of claim 1, wherein said means to provide said medicament dissolved or suspended in said propellant under positive airway pressure from said reservoir comprises a one-way valve, said one-valve configured to manipulate the quantity and pressure of said medicament dissolved or suspended in said propellant released from said reservoir.

5. An inhalation device comprising:
a. a reservoir containing a medicament dissolved or suspended in a propellant under pressure, said reservoir having an orifice;
b. an one-way electronic valve operably coupled to said orifice, said one-way electronic valve configured to manipulate the quantity and pressure of said medicament dissolved or suspended in said propellant released from said orifice, said one-way electronic valve configured to release said medicament dissolved or suspended in said propellant under positive airway pressure;
c. a narrow cylindrical tube mounted in fluid communication to said one-way electronic valve, said narrow cylindrical tube having a diameter substantially similar to lumen diameter of a human bronchi; said narrow cylindrical tube configured to provide said medicament dissolved or suspended in said propellant from said one-way valve into an oral or nasal cavity of a patient;
d. a control unit operably coupled to said one-way electronic valve permitting user manipulation of said one-way electronic valve;
e. a power module to supply power to said control unit and said one-way electronic valve.

6. The inhalation device of claim 5 wherein said inhalation device further comprises a mouthpiece, said mouthpiece juxtaposed over said narrow cylindrical tube and configured to be hold between lips of said patient.

7. The inhalation device of claim 5 wherein said inhalation device further comprises a nasal piece, said nasal piece juxtaposed over said narrow cylindrical tube and configured to be hold against the nasal opening.

8. The inhalation device of claim 5 wherein said narrow cylindrical tube is adapted to reduce the turbulence of said medicament dissolved or suspended in said propellant released from said one-way electronic valve providing substantial laminar flow of said medicament dissolved or suspended in said propellant.

9. The inhalation device of claim 5 wherein said medicament is one of: an anti-inflammatory substance, a vaccine, a bronchodilator.

10. An inhalation device comprising:
a. a reservoir containing a medicament dissolved or suspended in a propellant under pressure, said reservoir having an orifice;
b. an one-way mechanical valve operably coupled to said orifice in reservoir, said one-way mechanical valve configured to manipulate the quantity and pressure of said medicament dissolved or suspended in said propellant released from said orifice;
c. a narrow cylindrical tube mounted in fluid communication to said one-way mechanical valve, said narrow cylindrical tube having a diameter substantially similar to lumen diameter of a human bronchi; said narrow cylindrical tube configured to provide said medicament dissolved or suspended in said propellant from said one-way mechanical valve into an oral or nasal cavity of a patient, said narrow cylindrical tube is adapted to reduce the turbulence of said medicament dissolved or suspended in said propellant released from said one-way mechanical valve providing substantial laminar flow of said medicament dissolved or suspended in said propellant.
d. a control unit operably coupled to said one-way mechanical valve permitting user manipulation of said one-way mechanical valve.

11. The inhalation device of claim 10 wherein said inhalation device further comprises a mouthpiece, said mouthpiece juxtaposed over said narrow cylindrical tube and configured to be hold between lips of said patient.

12. The inhalation device of claim 10 wherein said inhalation device further comprises a nasal piece, said nasal piece juxtaposed over said narrow cylindrical tube and configured to be hold against the nasal opening.

13. The inhalation device of claim 10 wherein said reservoir is replaceable.

14. The inhalation device of claim 10 wherein said inhalation device is adapted for one-time use and is disposable.

15. The inhalation device of claim 10 wherein said control unit comprises a rotary dial having plurality of pressure resistance settings and adapted to be operated by said patient for setting desired positive airway pressure.
